(19) Europäisches Patentamt
European Patent Office
Office européen des brevets

(11) EP 3 936 131 A1

# (12) EUROPEAN PATENT APPLICATION

(43) Date of publication:
**12.01.2022 Bulletin 2022/02**

(21) Application number: **20185335.5**

(22) Date of filing: **10.07.2020**

(51) Int Cl.:
***A61K 31/505*** *(2006.01)* ***A61K 31/41*** *(2006.01)*
***A61K 31/4184*** *(2006.01)* ***A61K 31/4412*** *(2006.01)*
***A61K 31/4453*** *(2006.01)* ***A61K 31/4709*** *(2006.01)*
***A61K 31/4745*** *(2006.01)* ***A61K 31/496*** *(2006.01)*
***A61K 31/517*** *(2006.01)* ***A61K 31/519*** *(2006.01)*
***A61P 31/14*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Dompe' Farmaceutici S.P.A.**
**20122 Milan (IT)**

(72) Inventors:
- **BECCARI, Andrea Rosario**
  **80131 Napoli (IT)**
- **TALARICO, Carmine**
  **80131 Napoli (IT)**
- **MANELFI, Candida**
  **80131 Napoli (IT)**

(74) Representative: **Mauri, Elisabetta Maria Ester**
**Dompé Farmaceutici S.p.A.**
**Via Santa Lucia, 6**
**20122 Milano (IT)**

## (54) COMPOUNDS FOR THE TREATMENT OF ZIKA

(57) The present invention relates to compounds for use in the prevention or treatment of a Zika virus infection in a subject.

EP 3 936 131 A1

## Description

### FIELD OF THE INVENTION

The present invention relates to compounds useful for the prevention or treatment of Zika virus infections.

### STATE OF THE ART

Zika virus is a mosquito-borne flavivirus that is transmitted mainly by the bites of infected Aedes mosquitos, usually *Aedes aegypti,* the mosquito also responsible for the transmittion of dengue, chikungunya and yellow fever.

Zika virus can also transmitted from mother to fetus during pregnancy, and between different individuals through sexual contact, transfusion of blood and blood products, and organ transplantation.

Zika virus was first reported in Uganda in 1947 but outbreaks of infection were recorded only in recent years. The first recorded massive epidemics of Zika virus occured between 2007 and 2013 in subtropical and tropical areas of the Pacific islands.

More recently, in 2015, outbreaks and evidence of transmission appeared also throughout the Americas, Africa, and other regions of the world. To date, a total of 86 countries and territories have reported evidence of Zika infection.

The majority of people infected with Zika virus develop mild symptoms, such as fever, rash, conjunctivitis, muscle and joint pain, malaise, and headache, usually lasting for 2-7 days.

However, Zika virus in some cases can have severe complications.

In particular, it has been observed that Zika virus infection during pregnancy is a cause of microcephaly and other congenital abnormalities in the developing fetus and newborn. An unprecedented epidemic of congenital microencephalies and malformations was observed in the Americas during Zika 2015 outbreak. Zika infection in pregnancy may also result in pregnancy complications such as fetal loss, stillbirth, and preterm birth.

Furthermore, Zika virus infection has been found capable of triggering Guillain-Barre syndrome, neuropathy and myelitis, particularly in adults and older children.

At the moment, there is no treatment available for Zika virus infection or its associated diseases.

Therefore, the only approaches used for Zika infection are preventive measures against mosquito bites and the treatment of infected patients by supportive care.

It is therefore strongly needed the identification of drug compounds effective the treatment of patients infected by Zika virus.

Computational drug repurposing of already known drugs is an effective approach to find a rapid therapeutic solution for unmet medical needs (Vanhaelen, Q et al., Drug Discov. Today 22, 210-222(2017) (Karman, B&Sippl, Curr.Med.Chem.26, 5389-5409(2019).

### SUMMARY OF THE INVENTION

The inventors have analysed by Computer Associated Drug Design a library containing commercialized drugs and clinical candidates safe in man or characterized up to late clinical stage and have identified compounds potentially able to bind to viral proteins essential for Zika virus replication. The inventors have confirmed the activity of these compounds to inhibit Zika virus replication in infected host cells by *in vitro* experiments.

In details, the compounds identified as having inhibitory activity on viral are Rilpivirine hydrochloride, Posaconazole, Pyrvinium pamoate, Pranlukast hydrate, Candesartan cilexetil, Dactolisib, AEE-788, Olcegepant, AZD-1390, NVP-BGT226, Samotolisib and AZD-0156, better described in Table I below, wherein also the chemical name and formula for each compound are provided.

Table I

| N. | Chemical name | Current name | Chemical structure |
|---|---|---|---|
| 1 | 4-[4-[4-[(E)-2-Cyanovinyl]-2,6-dimethylphenylamino]pyrimidin-2-ylamino] benzonitrile hydrochloride | Rilpivirine HCl | 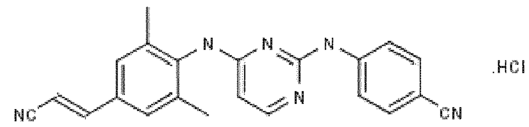 |
| 2 | 4-[4-[4-[4-[5(R)-(2,4-Difluorophenyl)-5-(1,2,4-triazol-1-ylmethyl)tetrahydrofuran-3(R)-ylmethoxy]phenyl]piperazin-1-yl]phenyl]-2-[1(S)-ethyl-2(S)-hydroxypropyl]-3,4-dihydro-2H-1,2,4-triazol-3-one | Posaconazole | 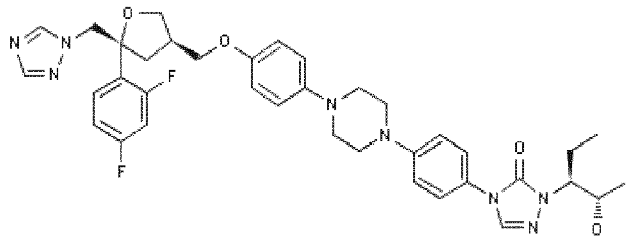 |
| 3 | 6-(Dimethylamino)-2-[2-(2,5-dimethyl-1-phenyl-1 H-pyrrol-3-yl)vinyl]-1-methylquinolinium hemi[4,4'-methylenebis(3-hydroxynaphthalen-2-carboxylic acid)] salt 6-(Dimethylamino)-2-[2-(2,5-dimethyl-1-phenyl-1 H-pyrrol-3-yl)vinyl]-1-methylquinolinium hemipamoate salt | Pyrvinium pamoate | 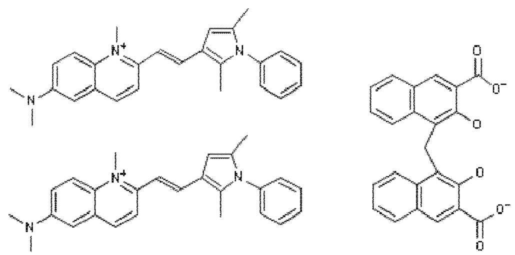 |
| 4 | N-[4-Oxo-2-(1H-tetrazol-5-yl)-4H-1-benzopyran-8-yl]-4-(4-phenylbutoxy) benzamide hemihydrate | Pranlukast hydrate | 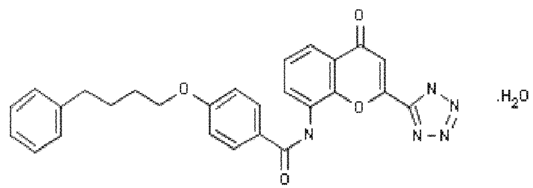 |
| 5 | (±)-2-Ethoxy-1-[2'-(1 H-tetrazol-5-yl)biphenyl-4-ylmethyl]-1 H-benzimidazole-7-carboxylic acid 1 -(cyclohexyloxycarbonyloxy) ethyl ester | Candesartan cilexetil | 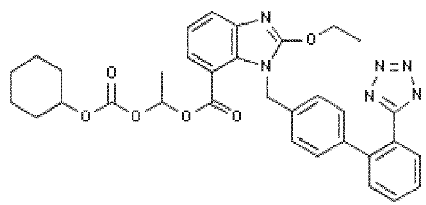 |

(continued)

| N. | Chemical name | Current name | Chemical structure |
|---|---|---|---|
| 6 | 2-Methyl-2-[4-[3-methyl-2-oxo-8-(3-quinolinyl)-2,3-dihydro-1 H-imidazo[4,5-c] quinolin-1-yl]phenyl]propanenitrile | Dactolisib | 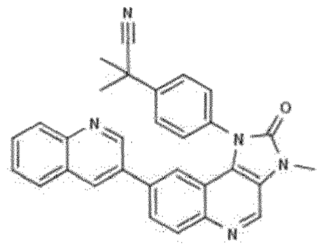 |
| 7 | 6-[4-(4-Ethylpiperazin-1-ylmethyl)phenyl]-N-[1(R)-phenylethyl]-7H-pyrrolo[2,3-d] pyrimidin-4-amine | AEE-788 | 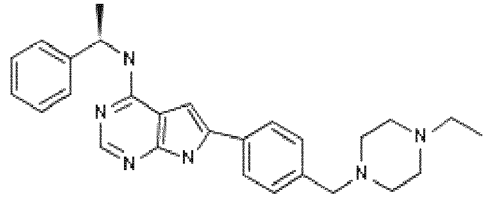 |
| 8 | N-[2-[5-Amino-1 (S)-[4-(4-pyridyl)piperazin-1-ylcarbonyl]pentylamino]-1(R)-(3,5-d ibromo-4-hyd roxybenzyl)-2-oxoethyl]-4-(2-oxo-1,2,3,4-tetrahydroquinazolin-3-yl)piperidine-1-carboxamide | Olcegepant | 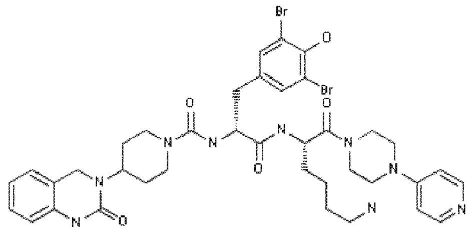 |
| 9 | 7-Fluoro-3-methyl-8-[6-[3-(piperidin-1-yl)propoxy]pyridin-3-yl]-1-(propan-2-yl)-1,3-dihydro-2H-imidazo[4,5-c]quinolin-2-one | AZD-1390 | 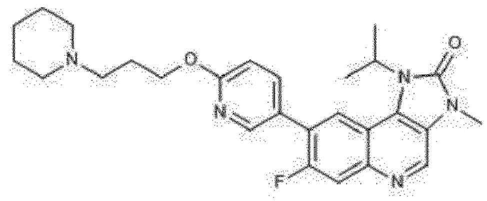 |

(continued)

| N. | Chemical name | Current name | Chemical structure |
|---|---|---|---|
| **10** | 8-(6-Methoxypyridin-3-yl)-3-methyl-1-[4-(1-piperazinyl)-3-(trifluoromethyl) phenyl]-2,3-dihydro-1H-imidazo[4,5-c]quinolin-2-one | NVP-BGT226 | 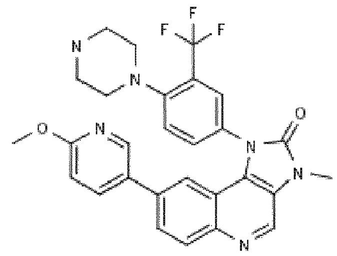 |
| **11** | 8-[5-(2-Hydroxypropan-2-yl)pyridin-3-yl]-1-[(2S)-2-methoxypropyl]-3-methyl-1,3-dihydro-2H-imidazo[4,5-c]quinolin-2-one | Samotolisib | 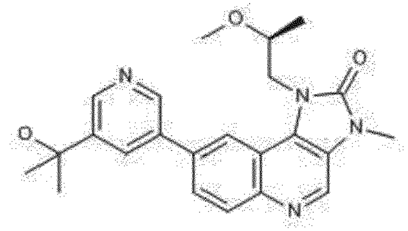 |
| **12** | 8-[6-[3-(Dimethylamino)propoxy]pyridin-3-yl]-3-methyl-1-(tetrahydro-2H-pyran-4-yl)-1,3-dihydro-2H-imidazo[4,5-c]quinolin-2-one | AZD-0156 | 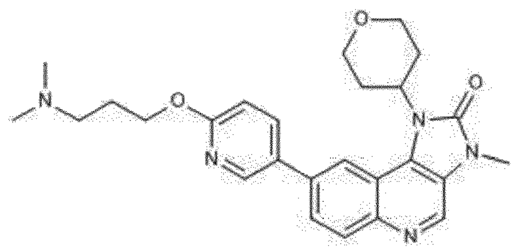 |

All these compounds are well known approved drugs or clinical candidates for different therapeutic indications with "safe in man" trials completed.

The experimental results obtained by the inventors and described in the Experimental Section demonstrate that these compounds are useful for the prevention and/or treatment of a Zika virus infection in a subject.

Accordingly, a first object of the invention is a compound selected from Rilpivirine hydrochloride, Posaconazole, Pyrvinium pamoate, Pranlukast hydrate, Candesartan cilexetil, Dactolisib, AEE-788, Olcegepant, AZD-1390, NVP-BGT226, Samotolisib and AZD-0156 for use in the prevention or treatment of Zika virus infection in a subject.

A second object of the invention is a pharmaceutical composition comprising i) a compound selected from Rilpivirine hydrochloride, Posaconazole, Pyrvinium pamoate, Pranlukast hydrate, Candesartan cilexetil, Dactolisib, AEE-788, Olcegepant, AZD-1390, NVP-BGT226, Samotolisib and AZD-0156 and ii) at least one inert pharmaceutically acceptable excipient, for use in the prevention or treatment of Zika virus infection in a subject

A third object of the invention is a method of preventing or treating a Zika virus infection in a subject, comprising diagnosing the subject as suffering from ot being at risk of developing a Zika virus infection and administering to said subject an effective amount of a compound selected from Rilpivirine hydrochloride, Posaconazole, Pyrvinium pamoate, Pranlukast hydrate, Candesartan cilexetil, Dactolisib, AEE-788, Olcegepant, AZD-1390, NVP-BGT226, Samotolisib and AZD-0156.

## FIGURES

Figure 1 shows viral load (expressed as $\log_{10}$ plaque forming equivalents) measured after treatment with vehicle or Rilpivirine hydrochloride, at concentrations of 30, 10 and 3 microM), as described in Example 2

Figure 2 shows viral load (expressed as $\log_{10}$ plaque forming equivalents) measured after treatment with vehicle or Posaconazole, at concentrations of 30, 10 and 3 microM, as described in Example 2

Figure 3 shows viral load (expressed as $\log_{10}$ plaque forming equivalents) measured after treatment with vehicle or Pyrvinium pamoate, at concentrations of 30, 10 and 3 microM, as described in Example 2

Figure 4 shows viral load (expressed as $\log_{10}$ plaque forming equivalents) measured after treatment with vehicle or Pranlukast hydrate, at concentrations of 30, 10 and 3 microM, , as described in Example 2

Figure 5 shows viral load (expressed as $\log_{10}$ plaque forming equivalents) measured after treatment with vehicle or Candesartan cilexetil, at concentrations of 30, 10 and 3 microM, as described in Example 2

Figure 6 shows viral load (expressed as $\log_{10}$ plaque forming equivalents) measured after treatment with vehicle or Dactolisib, at concentrations of 30, 10 and 3 microM, as described in Example 2

Figure 7 shows viral load (expressed as $\log_{10}$ plaque forming equivalents) measured after treatment with vehicle or AEE-788, at concentrations of 10 and 3 microM, as described in Example 2

Figure 8 shows viral load (expressed as $\log_{10}$ plaque forming equivalents) measured after treatment with vehicle or Olcegepant, at concentrations of 10 and 3 microM, as described in Example 2

## DETAILED DESCRIPTION OF THE INVENTION

A first object of the present invention is a compound selected from Rilpivirine hydrochloride, Posaconazole, Pyrvinium pamoate, Pranlukast hydrate, Candesartan cilexetil, Dactolisib, AEE-788, Olcegepant, AZD-1390, NVP-BGT226, Samotolisib and AZD-0156 or active derivatives thereof, for use in the prevention or treatment of a Zika virus infection in a subject.

The term "treatment" as used herein includes the curing, reduction, amelioration and/or stabiliziation of the infection and/or its symptoms.

The term "prevention" as used herein includes complete or partial inhibition of the infection and/or of its symptoms

As will be described in details in the experimental section, the present inventors have found that, in addition to their known activity, the above diverse compounds share the ability to inhibit Zika virus replication in infected host cells.

The selected compounds have the advantage that they have already been approved for clinical use or tested as safe in humans, in some cases are marketed drugs, and can therefore provide a rapid therapeutic approach to Zika virus infection.

Said subject may be a human or non human subject.

Preferably, said subject is a human subject.

According to a preferred embodiment, said subject is a subject that has been infected by Zika virus. Said infected subject may or may not have developed symptomatology of the disease.

According to an alternative preferred embodiment, said subject is at risk of being infected by Zika virus and said compound is used for the prevention of said infection.

According to a preferred embodiment, said subject is a pregnant woman. According to this embodiment, said compound is for use in the protection from Zika virus infection of the fetus of a pregnant woman infected or at risk of being infected with Zika virus.

According to the invention the active derivatives of the above identified compounds are derivatives of said compounds that maintain the pharmaceutical activity thereof. Preferably, said active derivatives are salts or prudrugs of the specifically identified compounds.

The compound for use according to the first object of the invention is preferably in form of a pharmaceutical composition where the compound is mixed with one or more pharmaceutically acceptable excipients.

Thus, a second object of the invention is a pharmaceutical composition comprising a compound selected from Rilpivirine hydrochloride, Posaconazole, Pyrvinium pamoate, Pranlukast hydrate, Candesartan cilexetil, Dactolisib, AEE-788, Olcegepant, AZD-1390, NVP-BGT226, Samotolisib and AZD-0156 or active derivatives therof, and at least one inert pharmaceutically acceptable excipient, for use in the prevention and/or treatment of a Zika virus infection in a subject, as above described.

The route of administration of the compound or pharmaceutical composition of the present invention depends on the specific compound used or contained in the pharmaceutical composition and is in accordance with known methods for administration of the compound, which is usually by systemic administration, preferably by oral, parenteral or inhalatory route. The term parenteral as used herein includes intravenous, intraperitoneal, intracerebral, intrathecal, intracranial, intramuscular, intraarticular, intrasynovial, intrasternal, intraocular, intraarterial, subcutaneous, intracutaneous injection or infusion techniques.

The composition of the present invention may be formulated into oral, inhalatory or injectable dosage forms such as tablets, capsules, powders, solutions, suspensions, and emulsions.

Preferably, the pharmaceutically acceptable excipient in the pharmaceutical composition includes any and all solvents, diluents, or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; cocoa butter and suppository waxes; oils such as peanut oil, cottonseed oi, safflower oil, sesame oil, olive oil, corn oil and soybean oil; glycols, such a propylene glycol; esters such as ethyl oleate and ethyl laurate; agar; buffering agents; alginic acid; pyrogen-free water; salts for regulating osmotic pressure; sterilized water; ethyl alcohol; preservatives, stabilizers, surfactants, emulsifiers, sweeteners, colorants, flavourings and the like.

The pharmaceutical composition of the present invention may be suitably formulated using appropriate methods known in the art or by the method disclosed in Remington's Pharmaceutical Science (recent edition), Mack Publishing Company, Easton Pa.

In one preferred embodiment, the invention provides for oral administration of the compound or the pharmaceutical composition of the invention

In such embodiment, the pharmaceutical composition is preferably in form of a tablet or capsule.

According to an alternative preferred embodiment, the compound or pharmaceutical composition of the invention is administered parenterally, preferably by intravenous administration or continuous infusion. This route of administration is particularly suitable for critically ill subjects

The amount of the compound according to the first object of the invention in the pharmaceutical composition of the present invention is the the amount usually employed for other indications of the specific compounds.

The amount can vary over a wide range depending on known factors, for example, the molecule used, the chosen route of administration and dosage form, the severity of the disease, the patient's age, body weight, general health status and sex, and the number of administrations per day. However, a person skilled in the art can determine the optimum amount in easily and routinely manner on the basis of the known dosage regime of the compound.

A third object of the invention is a method of preventing or treating a Zika virus infection in a subject, as abov defined, comprising diagnosing the subject as suffering from ot being at risk of developing a Zika virus infection and administering to said subject an effective amount of a compound selected from Rilpivirine hydrochloride, Posaconazole, Pyrvinium pamoate, Pranlukast hydrate, Candesartan cilexetil, Dactolisib, AEE-788, Olcegepant, AZD-1390, NVP-BGT226, Samotolisib and AZD-0156 or an active derivative therof.

The invention will be further described in the following examples, which do not limit the scope of the invention described in the claims.

**EXPERIMENTAL PART**

1. Computer Associated Drug Design

The EXSCALATE platform, the most powerful computing resources currently based in Europe to empower smart in-silico drug design, was exploited to perform High Performance Computing (HPC) simulations, with the final aim to select molecules active against the ZIKA virus. In particular docking simulations were conducted to virtual screen the Safe in Man (SIM) library, containing commercialized and under development drugs, already proved safe in man (> 10.000 drugs), against all the ZIKA proteins.

The simulation was performed using LiGen™ (Ligand Generator), the de novo structure based virtual screening software, designed and developed to run on HPC architectures, which represent the most relevant tool of the EXSCALATE platform. LiGen™ is formed by a set of tools that can be combined in a user-defined manner to generate project centric workflows. In particular, LiGenDock is a docking module using LiGenScore to compute the scoring function and the LiGenPass and LiGenPocket modules to obtain the 3D structure of the binding site. Both the docking algorithm implemented in LiGen™, the pharmacophoric docking (LiGenPh4) and the geometrical docking (LiGenGeodock), as well as the different scoring functions calculated, were used in this study to explore different protocols of Virtual Screening (VS) and select the best one in terms of performance.

The crystal structures of the ZIKA proteome, were obtained from the Protein Data Bank; Table 1 reports the list of the proteins analysed, with the corresponding PDB code.

Table1

| **Proteins** | **Sites** | **PDB code** |
|---|---|---|
| Protease (NS2B-NS3) | Orthosteric, allosteric | 5H6V, 5TFN |
| Helicase (NS3) | Orthosteric, allosteric, RNA | 5VI7, 5Y4Z |
| Methiltransferase (NS5-MT) | Orthosteric, allosteric, RNA | 5VIM, 5WXB, 5U0B |
| Polymerase (NS5-RdRp) | Orthosteric, allosteric, nucleosides | 5U0B, 5U0C |
| Envelope | Orthosteric | 5JHM |
| NS1 | Orthosteric | 5K6K |

More than one binding site was explored for each protein, in particular the orthosteric binding sites as well as allosteric known binding sites were identified for each protein and used to perform virtual screening. Overall, 26 sites were used considering that more than one crystal structure was used for each protein, selected among those available in the Protein Data Bank. An ensemble docking procedure was used to combine the results obtained with the different pdb structures for each of the 13 unique binding sites identified in the ZIKA proteins.

The SIM library is a Dompé dataset containing about 10.000 pharmaceutical compounds, corresponding to the list of drugs launched or under active development in several clinical phases derived from an Integrity database search (https://clarivate.com/cortellis/solutions/pre-clinical-intelligence-analytics/). All the compounds have been classified based on the mechanism of action in particular distinguishing the classes (antibacterial, antiviral, antiparasitic, antifungal, antinfective). Usually, the performance of a VS protocol is assessed by evaluating its capacity to recognize the active molecules among a large number of inactive decoys, where the active molecules represent about the 1% of the total number of compounds. The performances of the tested VS strategy were assessed by evaluating the capacity to correctly rank molecules, which are endowed with antiviral activity, and in particular Anti-Hepatitis C Virus Drugs, considering the high similarity between HCV and ZIKA proteins. Following this approach, about 110 molecules were labelled as active compounds.

The VS protocol performance was thus evaluated comparing the screening results of the SIM library on the ZIKA crystal structures, by using different docking algorithms and different scoring function. The conditions showing the best capacity to recognize the active molecules were used to prioritize the total list of screened molecules and select the top scored compounds, according to the score value (Csopt), that predicts the binding affinity of the molecules in the protein binding site. In particular the results of the virtual screening on the 13 binding sites of the six viral proteins reported in Table1 were combined, in order to select molecules with a potential poly-pharmacological effect on the ZIKA virus. To this aim, a total score, corresponding to the sum of the docking scores obtained for each protein, was calculated for each molecule and used to prioritize the most interesting molecules. The top scored molecules, in term of total_score, were the compounds Rilpivirine hydrochloride, Posaconazole, Pyrvinium pamoate, Pranlukast hydrate, Candesartan cilexetil, Dactolisib, AEE-788, Olcegepant, AZD-1390, NVP-BGT226, Samotolisib and AZD-0156, which were thus selected for further validation of the inhibitory activity in in vitro experiments.

2. In vitro screening of activity against ZIKV (Zika Virus)

The above compounds selected by the CADD analysis were tested in a ZIKV Vero CCL-81 cells (kidney epithelial cells from African green monkey) assay. This assay is based on viral infection of Vero cells followed by visual monitoring of cytopathic effects of the virus.

In details, the above compounds were diluted to the final concentrations of 30 microM, 10 microM and 3 microM. The final volume required for treatment was 200 microliters per well, in a total of 12 wells.

For infection of the cells, ZIKV (HS-2015-BA-01), isolated from a viremic patient with symptomatic infection in Bahia State, Brazil, in 2015, was used. The complete genome of the virus is under access KX520666 in Genbank. Viral dilutions were prepared in Multiplicity of Infection (MOI) at 0.1 according to a standardized method.

In particular, 37,9 microl of virus (6x107 pfu/mL) was used or a total of 25.000 microl (25 ml) RPMI-0% FBS medium

100 microliters of the viral dilution (MOI 0,1) were added to each well and incubated at 5% of CO2 at T= 37°C for 1 hour for adsorption step, swift rocking every 15'.

After discarding the supernatant, 200 microliters of diluted compounds at the different concentrations described above, or vehicle were added for each compound and left in incubation at 5% of CO2 at T= 37°C for 24 hours.

After 24 hours of the first treatment the treatment was repeated a second time, with the same concentration of each compound, and at the same conditions (5% of CO2 at T= 37°C for 1 hour).

After 24 hours, the supernatant was collected and used to perform MTT, LDH plaque titration assays, as described above.

• MTT Assay

For the MTT Assay, 10 microl of the MTT reagent (5mg/ml in PBS) were added to each well, so that a final concentration of 0.2 microg/mL per well was reached. Plate was incubated at T=37°C/5% CO2 for 90 minutes. After this period, cell supernatant was discarded, and methylsulphoxide (DMSO), 100 microl, was added per well and the plate was kept under constant agitation for 5 minutes. Reading was performed in a microplate reader at 540 nm. Cell viability was calculated according the formula:

$$\%\ \text{Cell viability} = [A \times 100 / (B)]$$

Where A corresponds to the absorbance value of treated cells and B corresponds to the absorbance value of untreated cells (mock).

• LDH (Lactate Dehydrogenase) Assay

The experiment was executed according to the protocol supplied by the manufacturer Bioclin Quibasa (REF K014): LACTATE DEHYDROGENASE LDH UV. 4 μL of the cell culture supernatant were taken from each well and transferred to a new 96-well plate. Then, 196 μL of LDH reagent was added, prepared in a 1: 5 ratio of reagent 1 (buffered substrate) plus reagent 2 (coenzyme). After adding the pre-prepared reagent, four absorbance readings (340 nm) were performed on a microplate reader as follows: first reading done right after the addition of the reagent, while the sequential readings were made 1, 3 and 4 minutes after the start of the measurement. Cell viability was calculated by using the formula:

$$\Delta/\text{min} = [(\text{Abs 1a. reading} - \text{Abs 1a. reading}) + (\text{Abs 3a. reading} - \text{Abs 4a. reading})] /2 \times 20794{,}34$$

where 20794, 34 corresponds to the correction factor provided by the supplier of the kit datasheet.

• Plaque titration assay

Vero CCL-81 cells (kidney epithelial cells from African green monkey) were seeded in 24-well plates at a density of 2 x cells/ well.

Cells were maintained in RPMI 1640 medium (Cultilab) supplemented with 10% FBS (Cultilab), 2 mM L-glutamine (Gibco) and 100U/50μg penicillin/streptomycin (Sigma-Aldrich) for 24 h at 37°C in a 5% CO2 incubator.

Serial ten-fold dilutions of viral suspension in sterile dilution tubes were prepared (10-1 up to 10-4). To this aim, tubes were filled with 450 microl of medium 0% SFB and 50 microl of the sample added on the first tube. The same procedure was repeated to obtain serial dilutions by passing 50 microliters to the next dilution and successively for the other dilutions. 300 μl of each serial dilution were added into each of the two wells of 24-well plate, maintaining two wells without infection.

The inoculated plate was incubated at 37°C for 1 hour in a 5% CO2 incubator to allow virus adsorption, swift rocking every 15'. Once discard the cell supernatant from all wells, 1 ml of 1,60% semi-solid carboxymethyl cellulose (CMC) overlay medium with 2% FBS was added to each well to limit the virus dissemination.

The plate was left 4 days at 37°C in a 5% CO2 incubator. Then cell monolayer was incubated with 1 ml of the 10% formol for a minimum 18 hours at room temperature for fixation. Once removed and fixed the overlay medium, the cell monolayer was washed in running water, stained with 1 ml of the 0.04% Violet crystal for 1 hour at room temperature, and the count of viral plaques to calculate plaque-forming unit (PFU) was conducted.

The results obtained in the MMT and LDH assays confirmed the absence of cell toxicity for all compounds at the tested concentrations.

The results obtained in the plaque titration assays are shown for Rilpivirine hydrochloride, Posaconazole, Pyrvinium pamoate, Pranlukast hydrate, Candesartan cilexetil, Dactolisib, AEE-788, Olcegepant, in Figures 1 to 8, respectively. As can be seen the addition of the tests compounds results in all cases in inhibition of virus replication and in reduction of consequent cytopathic effects of the virus compared to the control.

Similar results were obtained with the analogs of AZD-1390, NVP-BGT226, Samotolisib and AZD-0156.

**Claims**

1. A compound selected from Rilpivirine hydrochloride, Posaconazole, Pyrvinium pamoate, Pranlukast hydrate, Candesartan cilexetil, Dactolisib, AEE-788, Olcegepant, AZD-1390, NVP-BGT226, Samotolisib and AZD-0156 or active derivatives thereof, for use in the prevention or treatment of a Zika virus infection in a subject.

2. A compound for use as claimed in claim 1 wherein said subject is a human subject.

3. A compound for use in claim 1 or 2 wherein said subject has been infected by Zika virus.

4. A compound for use as claimed in caims 1 to 3 wherein said subject is at risk of being infected by Zika virus.

5. A compound for use as claimed in caims 1 to 4 wherein said subject is a pregnant woman.

6. A compound for use as claimed in claim 5, wherein said compound is for use in the protection from Zika virus infection of the fetus of a pregnant woman infected or at risk of being infected with Zika virus.

7. A compound for use as claimed in caims 1 to 6 wherein said compound is in a pharmaceutical composition in mixture with one or more pharmaceutically acceptable excipients.

8. A compound for use as claimed in caims 1 to 7, wherein said compound is administered orally.

9. A compound for use as claimed in caims 1 to 7, wherein said compound is administered parenterally, preferably by intraveneous administration or continuous infusion.

Fig. 1

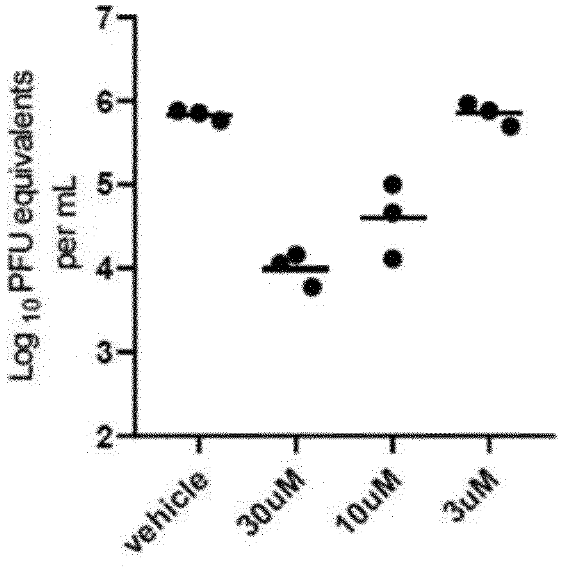
7
6
5
4
3
2
Log 10 PFU equivalents per mL
vehicle
30uM
10uM
3uM

Fig. 2

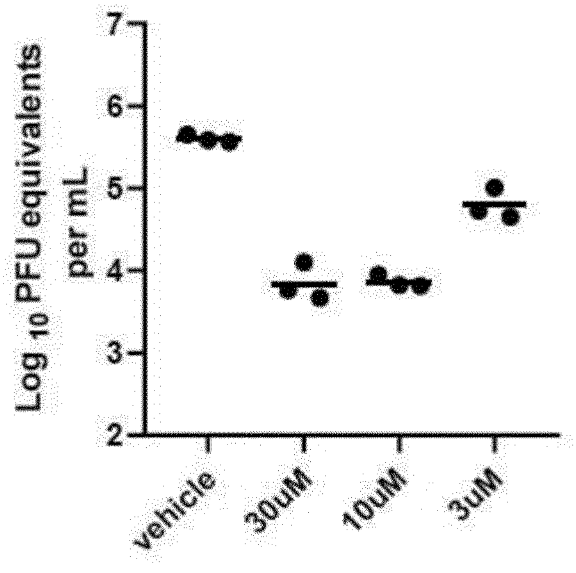
7
6
5
4
3
2
Log 10 PFU equivalents per mL
vehicle
30uM
10uM
3uM

Fig. 3

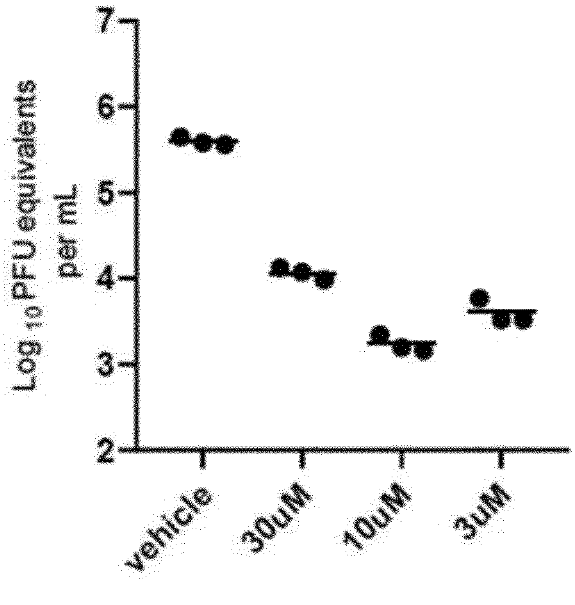
Log 10 PFU equivalents per mL
7
6
5
4
3
2
vehicle
30uM
10uM
3uM

Fig. 4

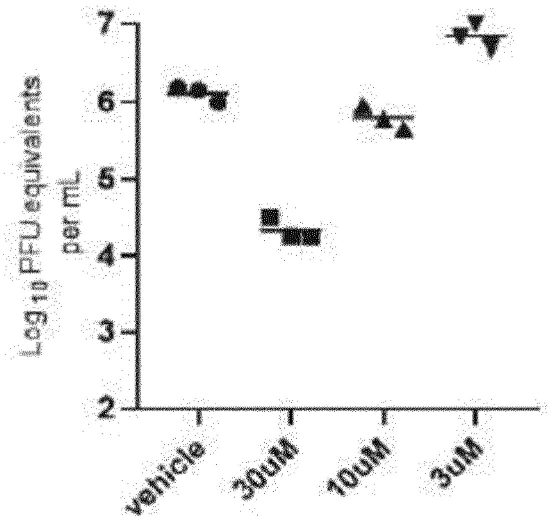
Log 10 PFU equivalents per mL
7
6
5
4
3
2
vehicle
30uM
10uM
3uM

Fig. 5

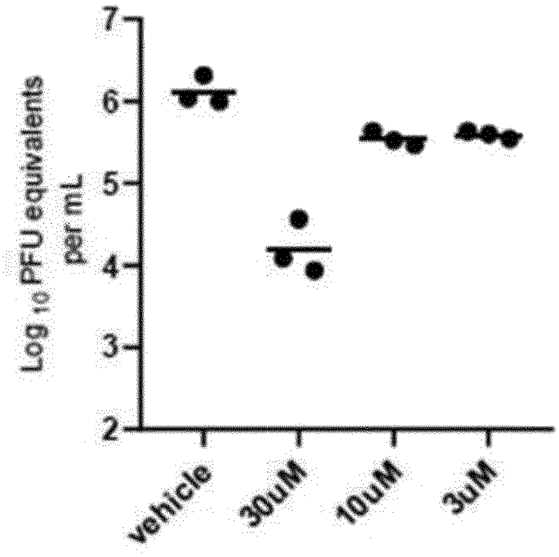
Log 10 PFU equivalents per mL
7
6
5
4
3
2
vehicle
30uM
10uM
3uM

Fig. 6

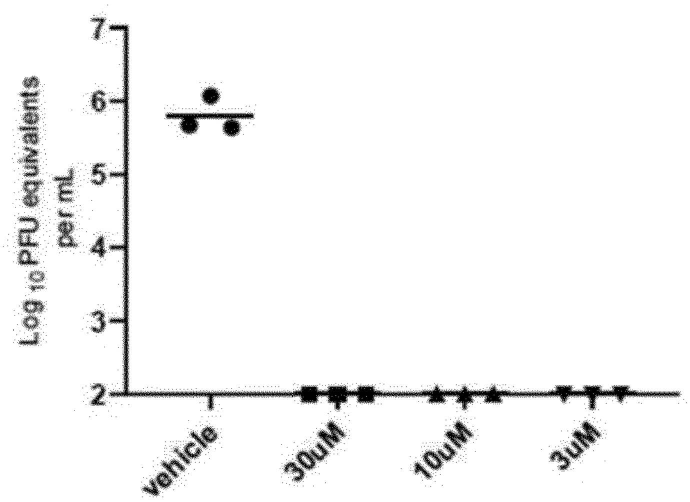
Log 10 PFU equivalents per mL
7
6
5
4
3
2
vehicle
30uM
10uM
3uM

Fig. 7

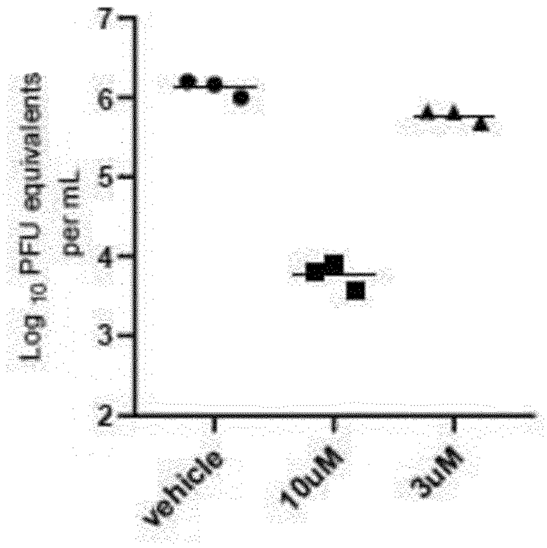
Log 10 PFU equivalents per mL
7
6
5
4
3
2
vehicle
10uM
3uM

Fig. 8

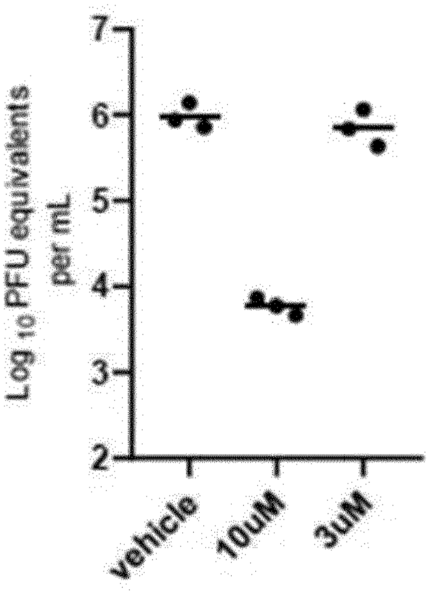
Log 10 PFU equivalents per mL
7
6
5
4
3
2
vehicle
10uM
3uM

Europäisches Patentamt
European Patent Office
Office européen des brevets

# EUROPEAN SEARCH REPORT

Application Number
EP 20 18 5335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2018/119371 A1 (UNIV TEMPLE [US]) 28 June 2018 (2018-06-28)<br>* paragraph [0060] - paragraph [0062]; claims; examples * | 1-4,7-9<br>1-9 | INV.<br>A61K31/505<br>A61K31/41<br>A61K31/4184<br>A61K31/4412<br>A61K31/4453<br>A61K31/4709<br>A61K31/4745<br>A61K31/496<br>A61K31/517<br>A61K31/519<br>A61P31/14 |
| X | LOE MARCUS WING CHOY ET AL: "Antiviral activity of the FDA-approved drug candesartan cilexetil against Zika virus infection",<br>ANTIVIRAL RESEARCH, ELSEVIER BV, NL,<br>vol. 172, 25 October 2019 (2019-10-25), XP085914487,<br>ISSN: 0166-3542, DOI: 10.1016/J.ANTIVIRAL.2019.104637<br>[retrieved on 2019-10-25]<br>* discussion;<br>paragraph [03.3] - paragraph [03.6] * | 1-9 | |
| X | MONTES-GRAJALES DIANA ET AL: "In silico drug repurposing for the identification of potential candidate molecules against arboviruses infection",<br>ANTIVIRAL RESEARCH, ELSEVIER BV, NL,<br>vol. 173, 28 November 2019 (2019-11-28), XP085948039,<br>ISSN: 0166-3542, DOI: 10.1016/J.ANTIVIRAL.2019.104668<br>[retrieved on 2019-11-28]<br>* discussion;<br>figures 5,6,7; table 2 * | 1-3 | TECHNICAL FIELDS SEARCHED (IPC)<br>A61K<br>A61P |

-/--

The present search report has been drawn up for all claims

| Place of search | Date of completion of the search | Examiner |
|---|---|---|
| Munich | 27 November 2020 | Venturini, Francesca |

1

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches Patentamt
European Patent Office
Office européen des brevets

# EUROPEAN SEARCH REPORT

Application Number
EP 20 18 5335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | "Abstracts from the Joint Meeting of the International Society for NeuroVirology (ISNV) and the Society on NeuroImmune Pharmacology (SNIP) April 10-14, 2018, Chicago, Illinois, USA", JOURNAL OF NEUROVIROLOGY, INFORMA HEALTHCARE, GB, vol. 24, no. Suppl 1, 19 March 2018 (2018-03-19), pages 1-102, XP037125875, ISSN: 1355-0284, DOI: 10.1007/S13365-018-0619-3 [retrieved on 2018-03-19]<br>* abstract P264 *<br>----- | 1,3,7,9<br><br>1-9 | |
| Y | MEUTIAWATI FEBRINA ET AL: "Posaconazole inhibits dengue virus replication by targeting oxysterol-binding protein", ANTIVIRAL RESEARCH, ELSEVIER BV, NL, vol. 157, 3 July 2018 (2018-07-03), pages 68-79, XP085439210, ISSN: 0166-3542, DOI: 10.1016/J.ANTIVIRAL.2018.06.017<br>* discussion *<br>----- | 1-9 | TECHNICAL FIELDS SEARCHED (IPC) |

The present search report has been drawn up for all claims

1

| Place of search | Date of completion of the search | Examiner |
|---|---|---|
| Munich | 27 November 2020 | Venturini, Francesca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 18 5335

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2020

| Patent document cited in search report | Publication date | Patent family member(s) | Publication date |
|---|---|---|---|
| WO 2018119371 A1 | 28-06-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **VANHAELEN, Q et al.** *Drug Discov. Today,* 2017, vol. 22, 210-222
- **KARMAN ; B&SIPPL.** *Curr.Med.Chem.,* 2019, vol. 26, 5389-5409
- Remington's Pharmaceutical Science. Mack Publishing Company